# EUROPEAN PATENT SPECIFICATION

(11) **EP 1 185 492 B1**
(45) Date of publication and mention of the grant of the patent: **25.05.2005**
(21) Application number: 00906477.5
(22) Date of filing: 24.02.2000
(51) Int. Cl.: C07C 41/09, C07C 41/50, C07C 1/20

(54) **ACID-CATALYSED REACTIONS**
SÄUREKATALYSIERTE UMSETZUNGEN
REACTIONS CATALYSEES PAR DES ACIDES

(30) Priority: 04.03.1999 GB 9904926
(43) Date of publication of application: 13.03.2002
(60) Divisional application: 04026835.1
(73) Proprietor: THOMAS SWAN AND CO., LTD., Consett, Co. Durham DH8 7ND (GB)
(72) Inventor: POLIAKOFF, Martyn, Nottingham NG7 2RD (GB); GRAY, William Keith, Nottingham NG7 2RD (GB); SWAN, Thomas Macklyn, Thomas Swan & Co. Ltd., County Durham DH8 7ND (GB); ROSS, Stephen Keith, Thomas Swan & Co. Ltd., Consett, County Durham DH8 7ND (GB); WIELAND, Stefan, 60311 Frankfurt (DE); ROEDER, Stefan, 60311 Frankfurt (DE)
(74) Representative: Atkinson, Jonathan David Mark
(86) International application number: PCT/GB2000/000673
(87) International publication number: WO 2000/051957

(56) References cited:
- EP-A- 0 652 202
- US-A- 5 831 116
- P. KRAMMER ET AL: "Untersuchungemn zum Synthesepotential in überkritischem Wasser" CHEMIE INGENIEUR TECHNIK, vol. 70, no. 12, 1998, pages 1559-1563, XP000791730 Weinheim
- GRAY, WILLIAM K. ET AL: "The Continuous Acid-Catalyzed Dehydration of Alcohols in Supercritical Fluids: A New Approach to the Cleaner Synthesis of Acetals, Ketals, and Ethers with High Selectivity" J. AM. CHEM. SOC. (1999), 121(46), 10711-10718 , XP000887316

## Description

The present invention relates to acid-catalysed reactions according to claim 1 for producing alkenes, ethers, acetals or ketals. Specifically the present invention relates to reactions for the formation of alkenes, ethers (including cyclic ethers), acetals and ketals in the presence of heterogeneous acid catalysts under supercritical conditions.

In particular, the present invention seeks to provide improved acid-catalysed reactions of alcohols (whether aliphatic, aromatic or heterocyclic) to produce ethers, alkenes, acetals or ketals as required.

The use of acid catalysis in industry is widespread and of importance.

We have shown that reactions of industrial importance such as ether, acetal, ketal and alkene formation can be carried out under supercritical conditions of temperature and pressure using a heterogeneous acid catalyst with significant advantages. Their use in supercritical fluids has not previously been described.

Using supercritical fluids as a solvent eliminates the need for conventional organic solvents and hence gives environmental benefits. However, a more important consequence is that the reactions can be modified by using supercritical fluids to give surprisingly high yields and/or selectivities.

US 5831116 discloses a process for partially oxidising alcohols to the corresponding either in supercritical carbon dioxide. The ether formed is symmetrical and there is no disclosure of the formation of unsymmetrical ethers or the formation of an ether from a polyol.

We have also found that certain products which are not easily accessible by conventional routes can be obtained more easily using the process of the present invention. Thus, whilst rearrangements may occur in conventional reactions we have found that certain reactions can be carried out under the conditions of the present invention without any significant rearrangement occurring. For example, ether formation in supercritical fluids under the conditions of the present invention can give rise to enhanced yields of *n*-alkyl ethers rather than the branched products which are obtained in conventional procedures.

### Ethers

Conventionally, the formation of ethers can be carried out via a wide number of classical routes e.g. Williamson Synthesis, dehydration of alcohols, alkylation of alcohols with inorganic esters, and alkylation with diazo compounds.

In the Williamson synthesis an alkyl halide is reacted with an alkoxide or aryloxide. This reaction therefore involves preforming the alkoxide from an alcohol by reaction with a strong base and subsequent reaction with an alkyl halide. This results in the generation of an equimolar amount of a halide salt which then must be disposed of. There are also the hazards associated with the handling of the alkyl halide in the case of volatile and/or toxic halides such as methyl iodide which is both a known carcinogen and very volatile.

In the case of reactions of alcohols with inorganic esters (e.g. the reaction of an alcohol with dimethyl sulfate), the inorganic esters are usually highly toxic. Again the alcohol has to be converted to the alkoxide using a strong base prior to the reaction. A further problem is the disposal of the aqueous effluent which can contain large amounts of inorganic salts.

The reaction of diazo compounds with alcohols is a cleaner reaction but suffers from the dangers associated with the thermal decomposition of diazo compounds. Also, diazo compounds are expensive and so it is not practicable to carry out this reaction on an industrial scale except for very high value products.

One of the most cost-effective and atom-efficient processes for producing ethers is the dehydration of alcohols using an acid catalyst. This reaction is usually carried out in the liquid phase but suffers the drawback that, in the reaction of n-alcohols, the alcohol can rearrange from the primary to secondary and then to the tertiary carbocation, thereby giving a mixture of products. A further problem is that the use of homogeneous catalysts such as sulphuric acid require a separation or neutralisation step at the end of the process. The use of homogeneous catalysts means that these processes are usually carried out in batch or semi-batch reactors. The use of batch systems also gives increased down time for charging and discharging. There is also the disadvantage that the product will be a mixture of thermodynamic and kinetic products due to relatively long residence times in the reactor. Distillation or some other physical means of separation is therefore usually required to separate the products.

A number of other reactions are also carried out to form ethers, usually on a smaller scale, such as the reaction of a Grignard reagent with an acetal or cyclisation of alcohols with lead, silver or mercury salts all of which suffer from high cost and problems of waste disposal.

### Acetals and Ketals

The most widely used method of formation of acetals and ketals is the reaction of alcohols with aldehydes or ketones under acidic conditions which involves the removal of water. These reactions are usually carried out in an excess of the alcohol or in an inert solvent. The conventional process therefore presents the problem of removing excess reagent and the catalyst must also be separated from the mixture if a homogenous catalyst is used.

### Alkenes

The industrial preparation of alkenes is normally carried out by dehydrogenation over noble metal catalysts or cracking reactions or dehydration reactions using inorganic acids. Such reactions present the problems of removal of catalyst, reaction solvent and distillation to purify products.

We have found that the use of supercritical fluids for the replacement of conventional solvents not only has significant environmental benefits but also leads to cleaner, higher yielding reactions.

Although the mechanism of the reactions is not fully understood it is believed that mass transport effects play a role in the observed improvements in yield and/or product selectivity. Surprisingly, the use of a heterogeneous catalyst according to claim 1 in place of a homogeneous catalyst under supercritical reaction conditions does not render the reaction ineffective. Instead we have found that the reactions benefit from improved yields and/or selectivity in product formation. This is so despite the expectation that the relatively severe conditions at or near the supercritical point of the reaction medium may give rise to a mixture of products because the reactants have sufficient energy to react via several different pathways.

One consequence of using the heterogeneous catalyst according to claim 1 is that there is no need for complicated separation procedures to liberate the product from the reaction mixture and catalyst. This represents quite a benefit in terms of both the time savings and cost savings of the present invention.

We have thus found that it is possible by using a combination of supercritical fluids and a heterogeneous catalyst according to claim 1 (e.g. the Deloxan ASP catalyst from Degussa or Acidic Amberlyst resin from Rohm and Haas) in a continuous flow reactor to carry out a number of reactions rapidly and cleanly. These reactions can often be performed in high yield and take place under supercritical conditions.

According to the present invention, there is provided a process in which reactants consisting only of an organic compound having the formula R¹CH₂OH, R¹R²CHOH, or R¹R²R³COH optionally in the presence of one or more further organic compounds having the formula R⁴CH₂OH, R⁵R⁶CHOH, R⁷R⁸R⁹COH or R¹⁰R¹¹CO, are exposed only to CO₂ and to a heterogenous catalyst which is able to provide a source of acid in a continuous flow reactor wherein the CO₂ is under supercritical conditions with the result that an ether, acetal, ketal or alkene product is formed, wherein the conditions of temperature, pressure, and flow rate are independently controlled, and wherein each of R¹ to R¹¹ is independently selected from: hydrogen, hydroxyl, or an optionally substituted alkyl, alkenyl, alkynyl, aralkyl, cycloalkyl, cycloalkenyl, aryl or heterocyclic group.

Aliphatic and aromatic alcohols are preferred because they give cleaner reactions, with aliphatic alcohols being most preferred on the grounds of ease of use and lower occurrence of side products.

It is preferred that each of R¹ to R¹¹ when present is an alkyl group which may be optionally substituted. Since the process of the present invention is applicable to diols, triols and higher alcohols as well as alcohols, it is particularly preferred that the optional substituent, when present, on one of the groups R¹ to R¹¹ is hydroxyl. It is also preferable, in order to avoid the risk of unwanted side products, that the total number of alcohol groups within the organic compound does not exceed three.

When any of the R¹ to R¹¹ groups are optionally substituted, the substituent groups which are optionally present may be any conventional substituent provided that any such substituent is not incompatible with alcohol functionality or with the reaction conditions.

Generally, when any of the R¹ to R¹¹ groups represents an optionally substituted alkyl group each group may independently be linear or branched and suitably contain 1 to 10, preferably 1 to 6 carbon atoms in the carbon chain, not including any optional substituent which may be present.

In relation to alkyl groups, specific examples of such optional substituents include halogen atoms and nitro, hydroxyl, C₁₋₄ alkyl, C₁₋₄ haloalkyl (especially CF₃), C₁₋₄ alkoxy, C₁₋₄ haloalkoxy and (C₁₋₄ alkoxy) carbonyl groups. Of these, hydroxyl and C₁₋₄ haloalkyl are preferred. It is preferred, however, that when any of groups R¹ to R¹¹ is alkyl that the alkyl moiety is unsubstituted.

In relation to a phenyl moiety, optional substituents include halogen atoms, and nitro, hydroxyl, C₁₋₄ alkyl, C₁₋₄ haloalkyl (especially CF₃) and C₁₋₄ alkoxy groups.

In general, 1 to 3 optional substituents may suitably be employed. Halogen atoms when present are preferably fluorine.

Where the starting materials for the reaction may exist in isomeric form the reaction of the present invention is applicable to all such optical or geometric isomers.

Suitable heterogeneous catalysts which are able to provide a source of said include Deloxan acid catalysts (Ex. Degussa-Hüls AG), zeolites, molecular sieves, clays, sulfonic acid derivatives, or other equivalent heterogeneous sources of a Brønsted acid (e.g. Amberlyst resin). The catalyst is ideally supported on an inert carrier. Preferably the catalyst contains sulfonic acid groups, and more preferably the catalyst is a Deloxan catalyst or an equivalent thereof.

The reactions of the present invention are preferably carried out in a single homogeneous phase. However, in reactions where water is generated this may separate out as a separate phase. This separation can be helpful to the reaction. The reactions are performed in the supercritical phase of CO₂.

When a fluid reaches its critical point its density is substantially decreased relative to its density at its boiling point at normal pressure. Small changes in pressure or temperature near the critical point cause additional changes in density.

The upper limit of temperature and pressure is governed only by limitations of the apparatus.

In practice, the choice of the supercritical fluid will depend upon the solubility of the organic compound in the fluid since a function of the supercritical fluid is to act as a solvent for the reagents. The medium is carbon dioxide.

Product formation may be monitored *in-situ* by means of IR spectroscopy using a suitably positioned IR cell, or by gas or liquid chromatography performed on samples drawn from the reactor periodically.

We are thus able to form ethers from primary and secondary alcohols with high conversion and good selectivity for *n*, or branched, products. Surprisingly *n*,*n* ethers can be formed by dehydration of *n*-alcohols with little or no rearrangement. In the case of phenolic compounds it is possible by altering the reaction conditions to favour ether formation over Friedel-Crafts alkylation and vice-versa.

We have also demonstrated that it is possible to form acetals and ketals under similar supercritical conditions as those employed for ether formation. In the case of branched alcohols, it is also possible to select conditions in which the corresponding alkene is obtained in high yield in preference to the ether product.

There is also little requirement for excess alcohols to be used in the process of the present invention because of the excellent conversion rates; this simplifies purification of the products.

The reactions are performed using a continuous flow reactor (preferably tubular reactor). It is therefore possible to control the residence time, and also the other reaction parameters independently. This allows greater control of the reaction resulting in more efficient and also more selective reactions than can be achieved in the conventional processes.

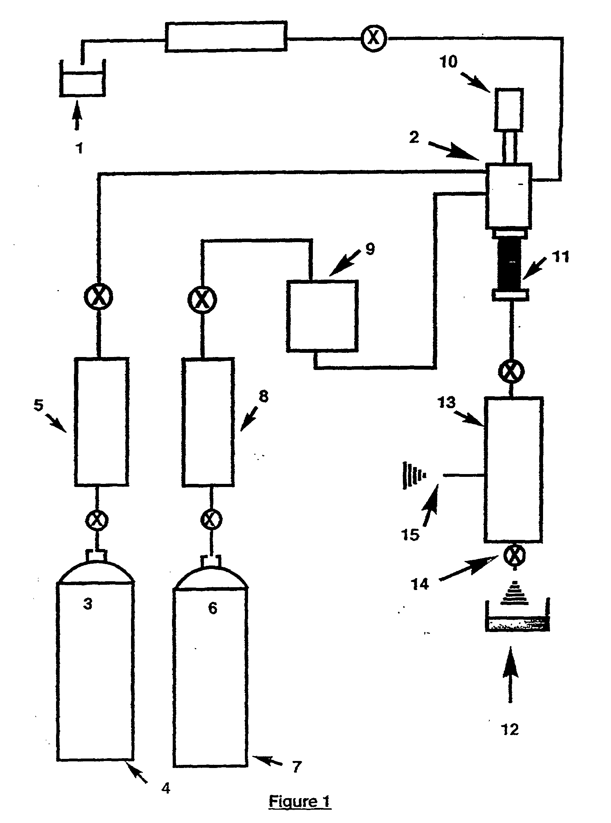

The present invention will now be described by way of example only with reference to Figure 1. Figure 1 is a schematic diagram of a continuous flow reactor according to the present invention.

The substrate 1, is pumped into mixer 2 which is a mixing vessel. The mixer 2 may include a stirrer (not shown). However, mixing of substrate 1 and fluid 3 may equally be effected without the use of stirrer. The substrate 1 is mixed with fluid 3 which is delivered from a reservoir via a pump to mixer 2. Where required, an additional reagent can be added via the same or another pump to mixer 2 with the ratio of the reagents being independently varied as required.

The temperature and the pressure in the mixer is adjusted to a temperature and pressure at or above the critical point of the fluid 3 as required.

Heating means are provided in the mixer for this purpose. The mixture is then passed into reactor 5 which contains a heterogeneous catalyst according to claim 1 (not shown) fixed on a suitable support. A means of controlling the pressure in the reactor is also included. The catalyst provides a source of Brønsted acid to the reaction mixture as the pressurised mixture passes over the catalyst.

After an appropriate mean residence time in reactor 5, the fluid 3, which now contains the product, is passed into pressure reduction unit 6. The products 7 are removed via a take-off tap after passing through pressure reduction unit 6. The flow rate of reactants through reactor 5 is controlled by a valve (not shown) in pressure reducer 6. Fluid 3 is vented through a relief pipe 8 for subsequent recycling or disposal.

The flow rate of the reagents is typically in the range of 0.5 to 20.0 ml/min. The reactor temperature is usually in the range of 30-350°C (again this will depend on the reaction medium) and the flow rate of supercritical fluid is usually in the range of 0.65 to 1.65 l/min of gaseous flow at atmospheric pressure-for a 10ml reactor.

The present invention will now be illustrated by the following examples in which the temperature is the catalyst bed temperature and the solvent flow is given as gaseous flow at atmosphere pressure.

### Example 1

### iso-Ether Formation

Isopropyl alcohol was exposed to a Deloxan ASP 1/7 acid catalyst (available from Degussa-Hüls AG) under the conditions given below in a continuous flow reactor. The volume of the reactor was 10 ml and the flow rate of the solvent was 0.65 L/min.

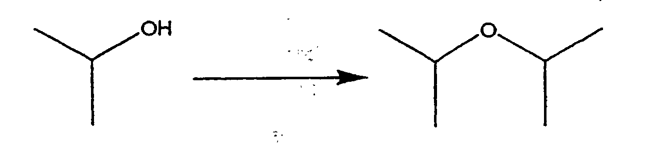

| Temp (°C) | Pressure (Bar) | Substrate Flow Rate(ml/min) | Solvent | Yield (%) |
|---|---|---|---|---|
| 200 | 200 | 0.5 | CO₂ | 29 |

### Example 2

### n-Ether Formation

*n*-Butanol was exposed to an Amberlyst 15 acid catalyst under the conditions given below in a continuous flow reactor. The volume of the reactor was 20 ml and the flow rate of the solvent was 0.65 L/min.

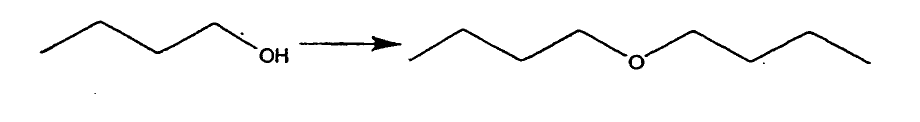

| Temp (°C) | Pressure (Bar) | Substrate Flow Rate (ml /min) | Solvent | Yield (%) |
|---|---|---|---|---|
| 200°C | 200 | 0.5 | CO₂ | 60 |

### Example 3

### Mixed Ether Formation

Isopropanol and *n*-propanol in the ratio of 1.2 parts isopropanol to 1.0 parts *n*-propanol were exposed to a Deloxan ASP 1/7 acid catalyst (Degussa-Hüls AG) under the conditions given below in a continuous flow reactor. The reactor volume was 20 ml and the flow rate of the solvent was 0.65 L/min.

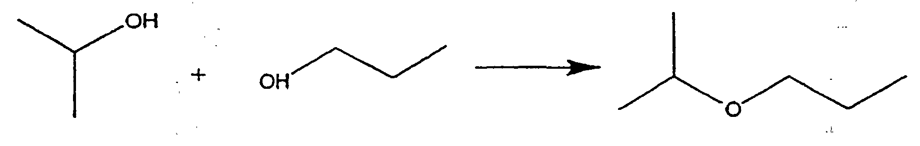

| Temp (°C) | Pressure (Bar) | Substrate Flow Rate(ml/min) | Solvent | Yield (%) |
|---|---|---|---|---|
| 150 | 200 | 0.5 | CO₂ | 41 |

### Example 4

### Cyclic Ether

1,4-butanediol was exposed to a Deloxan ASP 1/7 acid catalyst (available from Degussa-Hüls AG) under the conditions given below in a continuous flow reactor. The reactor volume was 10ml and the solvent flow rate was 0.65 L/min.

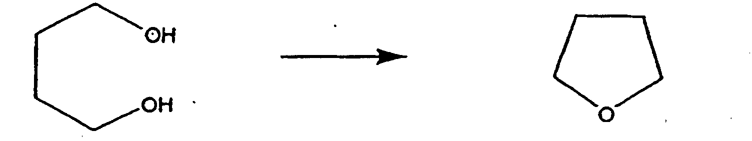

| Temp(°C) | Pressure (atm) | Substrate Flow Rate(ml/min) | Solvent | Yield (%) |
|---|---|---|---|---|
| 200 | 200 (2.03 × 10⁷Pa) | 0.5 | CO₂ | 100 |
| 150 | 100 (1.01 × 10⁷ Pa) | 0.5 | CO² | 100 |
| 125 | 100 (1.01 × 10⁷ Pa) | 0.5 | CO₂ | 87 |
| 200 | 100 (1.01 × 10⁷ Pa) | 2.0 | CO₂ | 93 |

### Example 5

### Mono-Etherification of Diols

1,6-Hexanediol and methanol in the ratio of 1.0 part 1,6 hexanediol to 1.1 part methanol were exposed to a Deloxan ASP 1/7 acid catalyst (Degussa-Hüls AG) under the conditions given below in a continuous flow reactor. The reactor volume was 10 ml and the flow rate of the solvent was 0.65 L/min.

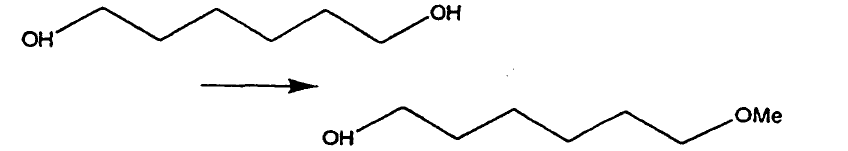

| Temp(°C) | Pressure (atm) | Substrate Flow Rate(ml/min) | Solvent | Yield (%) | Di-ether (%) |
|---|---|---|---|---|---|
| 100 | 200 (2.03 × 10⁷ Pa) | 0.5 | CO₂ | 32 | 13 |
| 200 | 200 (2.03 ×10⁷Pa) | 0.5 | CO₂ | 65 | 6 |

### Example 6

### Acetal Formation

1,2-Ethanediol and benzaldehyde in the ratio of 2.0 parts benzaldehyde to 1.0 part 1,2-ethanediol were exposed to a Deloxan ASP 1/7 acid catalyst (Degussa-Hüls AG) under the conditions given below in a continuous flow reactor. The reactor volume was 10 ml and the flow rate of solvent was 0.65 L/min.

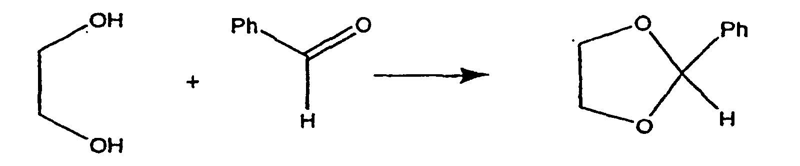

| Temp (°C) | Pressure (atm) | Substrate Flow Rate(ml/min) | Solvent | Yield (%) |
|---|---|---|---|---|
| 100 | 200 (2.03× 10⁷ Pa) | 0.5 | CO₂ | 89 |
| 200 | 200 (2.03× 10⁷ Pa) | 0.5 | CO₂ | 80 |

### Example 7

### Ketal Formation

1,2-Ethanediol and acetone in the ratio of 2.0 parts acetone to 1.0 part 1,2 ethanediol were exposed to a Deloxan c ASP 1/7 acid catalyst (Degussa-Hüls AG) under the conditions given below in a continuous flow reactor. The reactor volume was 10 ml and the flow rate of solvent was 0.65 L/min.

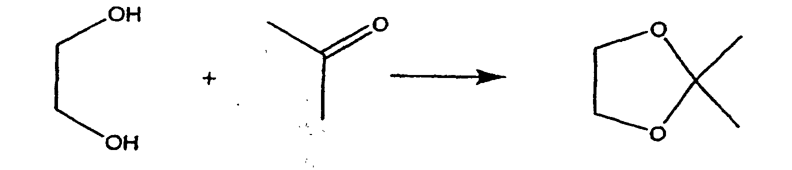

| Temp(°C) | Pressure (atm) | Substrate Flow Rate(ml/min) | Solvent | Yield (%) |
|---|---|---|---|---|
| 150 | 200 (2.03 × 10⁷ Pa) | 0.5 | CO₂ | 61 |

### Example 8

### Alkene Formation

2-Pentanol was exposed to a Deloxan ASP 1/7 catalyst (Degussa-Hüls AG) under the conditions given below in a continuous flow reactor. The reactor volume was 10ml and the flow rate of solvent was 0.65 L/min.

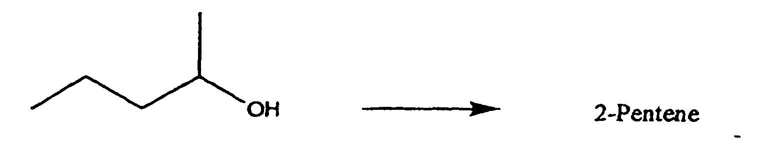

| Temp (°C) | Pressure (atm) | Substrate Flow Rate(ml/min) | Solvent | Pentene (%) |
|---|---|---|---|---|
| 200 | 200 (2.03 × 10⁷ Pa) | 0.5 | CO₂ | 100 |

## Claims

1. A process in which reactants consisting only of an organic compound having the formula R¹CH₂OH, R¹R²CHOH or R¹R²R³COH optionally in the presence of one or more further organic compounds having the formulae R⁴CH₂OH, R⁵R⁶CHOH, R⁷R⁸R⁹COH or R¹⁰R¹¹CO, are exposed only to CO₂ and to a heterogeneous catalyst which is able to provide a source of acid in a continuous flow reactor wherein the CO₂ is under supercritical conditions with the result that an ether, acetal, ketal or alkene product is formed, wherein the conditions of temperature, pressure, and flow rate are independently controlled, and wherein each of R¹ to R¹¹ is independently selected from: hydrogen or hydroxyl; an optionally substituted alkyl, alkenyl, alkynyl, aralkyl, cycloalkyl, cycloalkenyl, or aryl; or a heterocyclic group.

2. A process according to claim 1, wherein each of R¹ to R¹¹ when present is an optionally substituted alkyl group.

3. A process as claimed in claim 2 wherein each of the alkyl groups independently contains not more than 10 carbon atoms in the carbon chain excluding optional substituents if present.

4. A process according to claim 1 2 or 3, wherein the total number of alcohol groups within the organic compound does not exceed three.

5. A process according to any preceding claim, wherein the catalyst is selected from: zeolites, molecular sieves, clays, or sulfonic acid derivatives.

6. A process according to claim 5, wherein the catalyst is supported on an inert carrier.

7. A process according to claim 5 or 6, wherein the catalyst includes a promoter.

8. A process according to any of claims 5, 6 or 7, wherein the acidity of the catalyst is provided by a sulfonic acid group.

9. A process according to any preceding claim, wherein the reaction conditions are controlled in such a way that the products are selectively formed in high yield with insignificant rearrangement.

10. A process according to claim 9, wherein the reactant molecules are aliphatic and/or aromatic alcohols.

11. A process as claimed in claim 10, wherein the reactant molecules are aliphatic alcohols.

12. A process according to any preceding claim, in which the product is an ether.

13. A process according to claim 12, in which the reactant(s) and the product are straight-chain n-alkyl molecules.

14. A process according to claim 9 or 10, wherein the reaction conditions can be controlled in such a way that a particular alcohol is converted into an alkene in preference to an ether.

15. A process according to any preceding claim, in which the reactant(s) form a single homogeneous phase.

## Patentansprüche

1. Verfahren, in dem Reaktanten, die nur aus einer organischen Verbindung mit der Formel R¹CH₂OH, R¹R²CHOH oder R¹R²R³COH, gegebenenfalls in Anwesenheit einer oder mehrerer weiterer organischer Verbindungen mit den Formeln R⁴CH₂OH, R⁵R⁶CHOH, R⁷R⁸R⁹COH oder R¹⁰R¹¹CO, bestehen, nur CO₂ und einem heterogenen Katalysator, der eine Säurequelle bereitstellen kann, in einem kontinuierlichen Durchflussreaktor ausgesetzt werden, in dem das CO₂ unter superkritischen Bedingungen vorliegt, mit dem Ergebnis, dass ein Ether-, Acetal-, Ketal- oder Alken-Produkt gebildet wird, wobei die Bedingungen der Temperatur, des Druckes und des Durchsatzes unabhängig gesteuert werden und wobei jedes von R¹ bis R¹¹ unabhängig ausgewählt ist aus: Wasserstoff oder Hydroxyl; einem gegebenenfalls substituierten Alkyl, Alkenyl, Alkinyl, Aralkyl, Cycloalkyl, Cycloalkenyl oder Aryl; oder einer heterocyclischen Gruppe.

2. Verfahren nach Anspruch 1, in dem jedes von R¹ bis R¹¹, falls anwesend, eine gegebenenfalls substituierte Alkylgruppe ist.

3. Verfahren nach Anspruch 2, in dem jede der Alkylgruppen unabhängig nicht mehr als 10 Kohlenstoffatome in der Kohlenstoffkette enthält, ausschließlich fakultativer Substituenten, falls anwesend.

4. Verfahren nach Anspruch 1, 2 oder 3, in dem die Gesamtzahl an Alkoholgruppen in der organischen Verbindung drei nicht überschreitet.

5. Verfahren nach irgendeinem vorangehenden Anspruch, in dem der Katalysator ausgewählt ist aus: Zeolithen, Molekularsieben, Tonen oder Sulfonsäure-Derivaten.

6. Verfahren nach Anspruch 5, in dem der Katalysator auf einem inerten Träger getragen ist.

7. Verfahren nach Anspruch 5 oder 6, in dem der Katalysator einen Promotor einschließt.

8. Verfahren nach irgendeinem der Ansprüche 5, 6 oder 7, in dem die Acidität des Katalysators durch eine Sulfonsäure-Gruppe bereitgestellt wird.

9. Verfahren nach irgendeinem vorangehenden Anspruch, in dem die Reaktionsbedingungen auf solche Weise gesteuert werden, dass die Produkte selektiv in hoher Ausbeute mit unwesentlicher Umlagerung gebildet werden.

10. Verfahren nach Anspruch 9, in dem die Reaktantenmoleküle aliphatische und/oder aromatische Alkohole sind.

11. Verfahren nach Anspruch 10, in dem die Reaktantenmoleküle aliphatische Alkohole sind.

12. Verfahren nach irgendeinem vorangehenden Anspruch, in dem das Produkt ein Ether ist.

13. Verfahren nach Anspruch 12, in dem der oder die Reaktant(en) und das Produkt geradkettige n-Alkyl-Moleküle sind.

14. Verfahren nach Anspruch 9 oder 10, in dem die Reaktionsbedingungen auf solche Weise gesteuert werden können, dass ein spezieller Alkohol bevorzugt in ein Alken anstelle eines Ethers überführt wird.

15. Verfahren nach irgendeinem vorangehenden Anspruch, in dem der oder die Reaktant(en) eine einzige homogene Phase bilden.

## Revendications

1. Procédé dans lequel des réactifs consistant uniquement en un composé organique ayant pour formule R¹CH₂OH, R¹R²CHOH ou R¹R²R³COH, éventuellement en la présence d'un ou de plusieurs autres composés organiques ayant pour formule R⁴CH₂OH, R⁵R⁶CHOH, R⁷R⁸R⁹COH ou R¹⁰R¹¹CO, sont exposés uniquement à du CO₂ et à un catalyseur hétérogène qui est apte à fournir une source d'acide dans un réacteur à écoulement continu dans lequel le CO₂ est à l'état supercritique entraînant la formation d'un produit éther, acétal, cétal ou alcène, les conditions de température, de pression et de débit étant contrôlées indépendamment et chacun de R¹ à R¹¹ étant choisi indépendamment les uns des autres parmi : hydrogène ou hydroxyle ; un groupe alkyle, alcényle, alcynyle, aralkyle, cycloalkyle, cycloalcényle ou aryle éventuellement substitué ; ou un groupe hétérocyclique.

2. Procédé selon la revendication 1, dans lequel chacun de R¹ à R¹¹, lorsqu'il est présent, est un groupe alkyle éventuellement substitué.

3. Procédé selon la revendication 2, dans lequel chacun des groupes alkyle, indépendamment les uns des autres, ne contient pas plus de 10 atomes de carbone dans la chaîne carbonée, non compris les éventuels substiluants s'ils sont présents.

4. Procédé selon la revendication 1, 2 ou 3, dans lequel le nombre total des groupes alcool à l'intérieur du composé organique ne dépasse pas trois.

5. Procédé selon l'une quelconque des revendications précédentes, dans lequel le catalyseur est choisi parmi : zéolites, tamis moléculaires, argiles, ou dérivés de l'acide sulfonique.

6. Procédé selon la revendication 5, dans lequel le catalyseur est supporté sur un support inerte.

7. Procédé selon la revendication 5 ou 6, dans lequel le catalyseur comprend un promoteur.

8. Procédé selon l'une quelconque des revendications 5, 6 ou 7, dans lequel l'acidité du catalyseur est apportée par un groupe acide sulfonique.

9. Procédé selon l'une quelconque des revendications précédentes, dans lequel les conditions réactionnelles sont réglées de telle manière que les produits sont sélectivement formés en un haut rendement avec un réarrangement négligeable.

10. Procédé selon la revendication 9, dans lequel les molécules réactives sont des alcools aliphatiques et/ou aromatiques.

11. Procédé selon la revendication 10, dans lequel les molécules réactives sont des alcools aliphatiques.

12. Procédé selon l'une quelconque des revendications précédentes, dans lequel le produit est un éther.

13. Procédé selon la revendication 12, dans lequel le ou les réactifs et le produit sont des molécules n-alkyle à chaîne linéaire.

14. Procédé selon la revendication 9 ou 10, dans lequel les conditions réactionnelles peuvent être contrôlées de manière telle qu'un alcool particulier est converti en un alcène de préférence à un éther.

15. Procédé selon l'une quelconque des revendications précédentes, dans lequel le ou les réactifs forment une phase homogène unique.
